# EUROPÄISCHE PATENTSCHRIFT

(11) **EP 2 670 457 B1**
(45) Veröffentlichungstag und Bekanntmachung des Hinweises auf die Patenterteilung: **07.03.2018**
(21) Anmeldenummer: 12702426.3
(22) Anmeldetag: 27.01.2012
(51) Int. Cl.: A61M 5/145

(54) **DOSIERVORRICHTUNG FÜR EINE MEDIZINISCHE VERWENDUNG, MIT WENIGSTENS EINER EINRICHTUNG ZUM FÜHREN WENIGSTENS EINES KABELS**
MEDICAL-TECHNICAL DOSING DEVICE COMPRISING A DEVICE FOR GUIDING A CABLE
UN DISPOSITIF DE DOSAGE MÉDICO-TECHNIQUE AVEC UN ÉLÉMENT PERMETTANT DE GUIDER UN CÂBLE

(30) Priorität: 31.01.2011 DE 102011009909; 31.01.2011 US 201161437699 P
(43) Veröffentlichungstag der Anmeldung: 11.12.2013
(73) Patentinhaber: Fresenius Medical Care Deutschland GmbH, 61352 Bad Homburg (DE)
(72) Erfinder: OESTERREICH, Stefan, 61267 Neu-Anspach (DE)
(74) Vertreter: Bobbert & Partner Patentanwälte PartmbB
(86) Internationale Anmeldenummer: PCT/EP2012/000368
(87) Internationale Veröffentlichungsnummer: WO 2012/104043

(56) Entgegenhaltungen:
- EP-A1- 1 066 846
- WO-A1-99/54651
- US-A1- 2004 020 675
- US-A1- 2005 234 382
- US-A1- 2008 306 443

## Beschreibung

Die vorliegende Erfindung betrifft eine Dosiervorrichtung gemäß Anspruch 1 sowie eine Behandlungsvorrichtung gemäß Anspruch 7.

In medizinischen Vorrichtungen werden regelmäßig Bauteile verwendet, die eine externe Spannungsversorgung oder eine leitergebundene Signalverbindung zu einem Äußeren der Vorrichtung benötigen. Diese Signal- oder Spannungsverbindung wird in vielen Fällen mittels Kabel bereitgestellt.

Eine Aufgabe der vorliegenden Erfindung ist es, eine Dosiervorrichtung mit wenigstens einer Einrichtung zum Führen wenigstens eines Kabels oder anderweitig ausgestalteten Leiters, im Folgenden auch kurz erfindungsgemäße Einrichtung genannt, vorzuschlagen.

Die erfindungsgemäße Aufgabe wird durch eine Dosiervorrichtung mit den Merkmalen des Anspruchs 1 gelöst.

Die erfindungsgemäße Einrichtung ist mit wenigstens einem bewegbaren Abschnitt der Dosiervorrichtung mitführbar verbunden, alternativ ist die erfindungsgemäße Einrichtung für eine mitführbare Verbindung hiermit vorgesehen.

Die Verbindung ermöglicht ein Mitbewegen der Einrichtung mit dem bewegbaren Abschnitt der Dosiervorrichtung. Dadurch werden die Einrichtung und der bewegbare Abschnitt gemeinsam bewegt, zumindest sind sie für eine derartige gemeinsame Bewegung vorgesehen.

Die erfindungsgemäße Dosiervorrichtung weist die Merkmale des Anspruchs 1 auf.

Die erfindungsgemäße Behandlungsvorrichtung weist die Merkmale des Anspruchs 7 auf.

Die erfindungsgemäße Behandlungsvorrichtung weist wenigstens eine erfindungsgemäße Dosiervorrichtung auf oder ist hiermit verbunden.

Vorteilhafte Weiterentwicklungen der vorliegenden Erfindung sind jeweils Gegenstand von Unteransprüchen und Ausführungsformen.

Erfindungsgemäße Ausführungsformen können eines oder mehrere der im Folgenden genannten Merkmale aufweisen.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll bestimmte erfindungsgemäße Ausführungsformen erläutern.

Ein Kabel ist in bestimmten Ausführungsformen der vorliegenden Erfindung ein Leiter. Beide Begriffe sind im Folgenden synonym zu verstehen, wo immer dies für den Fachmann Sinn ergibt.

In manchen erfindungsgemäßen Ausführungsformen ist das Kabel oder der Leiter ein elektrischer Leiter zum Versorgen von elektrischen Bauteilen mit Spannung. Der Leiter kann ein- oder mehradrig sein. Der Leiter kann eine Litze sein oder aufweisen.

In bestimmten Ausführungsformen ist der elektrische Leiter mit einem Isolator ummantelt, beispielsweise mit einer Kunststoffummantelung oder einer Isolierhülle.

In bestimmten Ausführungsformen dient das Kabel zur Signalübertragung, beispielsweise zur Übertragung digitaler Spannungssignale.

In bestimmten erfindungsgemäßen Ausführungsformen ist das Kabel ein optischer Leiter oder weist zumindest einen solchen auf.

Der Begriff "Führen eines Kabels", wie er hierin in bestimmten Ausführungsformen verwendet wird, bezeichnet das Anordnen oder Verlegen eines Kabels, wie es beispielsweise in Kabelschächten oder Kabelkanälen erfolgt.

Der Begriff "Führen eines Kabels", wie er hierin in manchen Ausführungsformen verwendet wird, bezeichnet das Lagern, Aufnehmen, Abstützen oder Stützen des Kabels.

Der Begriff "Führen eines Kabels", wie er hierin in einigen Ausführungsformen verwendet wird, bezeichnet das Mitnehmen des Kabels in einer Bewegung, welche die erfindungsgemäße Einrichtung ausführt.

Eine medizintechnische Dosiervorrichtung ist in bestimmten Ausführungsformen eine Vorrichtung zur Dosierung von Flüssigkeiten, Medikamentenlösungen, Infusionen, usw.

In bestimmten Ausführungsformen weist die erfindungsgemäße Dosiervorrichtung wenigstens eine Aufnahmekammer für die zu dosierende Substanz oder Lösung, z. B. in Form einer in ihrem Volumen veränderliche Spritze, eine Halteeinrichtung für die Aufnahmekammer, einen bewegbaren Abschnitt sowie wenigstens eine erfindungsgemäße Einrichtung zum Führen wenigstens eines Kabels auf.

In manchen Ausführungsformen weist die erfindungsgemäße medizintechnische Dosiervorrichtung zudem wenigstens ein elektrisches Bauteil - wie z. B. einen spannungsbetriebenen Sensor - und/oder ein Stellelement zum Bewegen des bewegbaren Abschnitts auf.

Das Stellelement ist in bestimmten erfindungsgemäßen Ausführungsformen eine Zugrohr. In manchen erfindungsgemäßen Ausführungsformen dreht sich das Stellelement um seine eigene Längsachse beim Bewegen des bewegbaren Abschnitts.

Die Aufnahmekammer ist in manchen Ausführungsformen ein - in den meisten Fällen zylindrischer - Hohlraum mit einem darin bewegbar angeordneten Kolben, auch Stempel genannt, sowie einer Düse oder Öffnung zum Ausbringen des Inhalts der Aufnahmekammer. Die Befüllung der Aufnahmekammer kann auf unterschiedliche Weise erfolgen - sie kann vom Arzt befüllt werden oder werkseitig befüllt sein.

In manchen Ausführungsformen ist die Aufnahmekammer aus Kunststoff hergestellt.

Die Aufnahmekammer kann eine herkömmliche Medikamentenspritze sein.

Die Aufnahmekammer kann als Einmalprodukt ("Disposable") ausgestaltet sein. Alternativ ist sie zur wiederholten Verwendung vorgesehen.

In manchen Ausführungsformen ist die Düse oder Öffnung zum Ausbringen des Aufnahmekammerinhalts mit einem Schraubgewinde ausgestaltet. Das Schraubgewinde kann vorgesehen sein, um einen Schlauch (z. B. Infusions-Schlauch), eine Hohlnadel (Kanüle), einen Katheter, Dreiwegehähne oder ähnliches hieran anzuschließen. Das Schraubgewinde kann als genormtes Verbindungssystem (insbesondere als "Luer-Lock") ausgestaltet sein.

In manchen Ausführungsformen ist der bewegbare Abschnitt der Dosiervorrichtung mit dem bewegbaren Kolben der Aufnahmekammer verbunden, oder für eine solche Verbindung vorgesehen.

Der bewegbare Abschnitt ist in bestimmten Ausführungsformen als Griffstück ausgestaltet.

Das Stellelement und/oder die erfindungsgemäße Einrichtung ist bzw. sind in bestimmten erfindungsgemäßen Ausführungsformen mit dem bewegbaren Abschnitt der Dosiervorrichtung verbunden oder zumindest für eine solche Verbindung vorgesehen.

Die Verbindung zwischen Stellelement und/oder erfindungsgemäßer Einrichtung einerseits und bewegbarem Abschnitt andererseits kann lösbar oder nicht lösbar ausgestaltet sein.

Das Stellelement kann den bewegbaren Abschnitt, z. B. mithilfe eines Mitnehmers oder mittels einer entsprechenden Verbindung, bewegen. Auf diese Weise kann z. B. der Kolben der Aufnahmekammer durch das Stellelement bewegt werden, um den Aufnahmekammerinhalt über die Düse auszubringen.

In manchen erfindungsgemäßen Ausführungsformen ist das Stellelement des bewegbaren Abschnitts mit einem Motor, z. B. einem Schrittmotor, einem Getriebe oder mit einer anderen Stelleinheit, die mittels einer Steuerung oder Regelung gekoppelt sein kann, verbunden.

In bestimmten erfindungsgemäßen Ausführungsformen ist das Kabel mit dem elektrischen Bauteil der Dosiervorrichtung verbunden oder weist das elektrische Bauteil auf.

Ein elektrisches Bauteil kann ein Sensor, Schaltkreis, Optokoppler oder ähnliches sein.

In manchen Ausführungsformen ist die erfindungsgemäße Einrichtung über ihre gesamte Länge oder in wenigstens einem Abschnitt hiervon ein Kabelkanal.

In bestimmten Ausführungsformen weist die erfindungsgemäße Einrichtung über ihre gesamte Länge oder in wenigstens einem Abschnitt hiervon eine runde, rechteckige, ovale oder jede andere Querschnittsform auf, insbesondere eine geschlossene Querschnittsform.

Die erfindungsgemäße Einrichtung kann aus Kunststoff, Metall, faserverstärkten Materialien oder aus anderen Materialien hergestellt sein oder solche aufweisen.

Die erfindungsgemäße Einrichtung kann gerade sein und/oder einmal oder mehrfach gebogen sein, oder sie kann eine andere Form haben.

In bestimmten Ausführungsformen ist die erfindungsgemäße Einrichtung mit dem bewegbaren Abschnitt der Dosiervorrichtung zur gemeinsamen Bewegung mitbewegbar verbunden oder zu einer solchen Verbindung vorgesehen.

In manchen Ausführungsformen ist die erfindungsgemäße Einrichtung in einer Art Mitnehmerfunktion mit dem bewegbaren Abschnitt verbunden. Eine Mitnehmerfunktion kann beispielsweise eine nicht dauerhaft formschlüssige Verbindung sein, z. B. ausgebildet als ein Zapfen oder ein Ring an der erfindungsgemäßen Einrichtung, der z. B. bei einer Linearbewegung des bewegbaren Abschnitts von diesem mitgeführt wird.

In bestimmten Ausführungsformen der Erfindung ist die erfindungsgemäße Einrichtung dauerhaft mit dem bewegbaren Abschnitt der Dosiervorrichtung verbunden oder zu einer solchen Verbindung vorgesehen.

Der Begriff "dauerhaft" bezeichnet in diesen Ausführungsformen der vorliegenden Erfindung einen begrenzten oder unbegrenzten Zeitraum.

Ein begrenzter Zeitraum kann beispielsweise einen Tag, mehrere Tage, Wochen, Monate oder Jahre betragen.

Ein begrenzter Zeitraum kann beispielsweise von der Inbetriebnahme bis zu einem nächsten Wartungszeitpunkt betragen. Ein begrenzter Zeitraum kann auch vom erstmaligen Einsetzen eines elektrischen Bauteils, und damit verbunden dem erstmaligen Verwenden des Kabels in der Dosiervorrichtung, bis zum Austausch oder Ersatz dieses Bauteils zu einem späteren Zeitpunkt betragen.

Eine dauerhafte Verbindung kann wieder lösbar oder nicht lösbar ausgestaltet sein. Eine lösbare Verbindung ist beispielsweise mittels eines Einsteckens (z. B. Presspassung) der Einrichtung in den bewegbaren Abschnitt der Dosiervorrichtung, eines Verschraubens oder eines wieder lösbaren Klebens erzielbar.

Eine Verbindung kann mit dem Fachmann bekannten Verfahren gegen Flüssigkeiten und/oder Gase abgedichtet werden.

In bestimmten erfindungsgemäßen Ausführungsformen ist das Kabel mit einem in der Dosiervorrichtung fixierten Sensor verbunden, welcher vorgesehen und/oder geeignet ist, das Vorhandensein und/oder die Lage wenigstens des bewegbaren Abschnitts und/oder der Aufnahmekammer innerhalb der Dosiervorrichtung zu erfassen.

Der Sensor kann ein aktiver oder passiver Sensor sein.

In manchen erfindungsgemäßen Ausführungsformen ist der Sensor ein Ultraschallsensor, ein optoelektronischer Sensor, ein kapazitiver Sensor oder ein anderer Sensor. Auch auf anderen Messprinzipien basierende Sensoren sind erfindungsgemäß umfasst, beispielsweise magnetisch-induktive Sensoren. Der zu detektierende Abschnitt der erfindungsgemäßen Einrichtung oder Dosiervorrichtung kann entsprechend vorbereitet oder ausgestaltet sein. So kann z. B. die Aufnahmekammer Materialien enthalten, die mit magnetisch-induktiven Sensoren detektiert werden können.

In manchen erfindungsgemäßen Ausführungsformen erfolgt die Überprüfung der Lage bzw. Position mittels des Sensors durch einen Vergleich von aktuell erhaltenen Sensorsignalen mit Referenzsignalen.

Die erfindungsgemäße Einrichtung ist in eine Verbindungsöffnung der Dosiervorrichtung eingeführt, oder ist dafür vorgesehen eingeführt zu werden.

Die Verbindungsöffnung liegt in einigen erfindungsgemäßen Ausführungsformen in einem Gehäuse - oder einen Abschnitt hiervon - der Dosiervorrichtung.

Die Verbindungsöffnung verbindet in manchen erfindungsgemäßen Ausführungsformen ein Inneres der Dosiervorrichtung mit einem Äußeren der Dosiervorrichtung oder mit deren Umgebung.

Die Verbindungsöffnung hat insgesamt oder zumindest in einem Abschnitt hiervon in bestimmten erfindungsgemäßen Ausführungsformen einen runden Querschnitt. Der Querschnitt kann jedoch auch oval oder rechteckig sein, oder jede andere Form aufweisen.

Die Einrichtung wird in die Verbindungsöffnung der Dosiervorrichtung eingeschoben, eingesteckt oder in anderer Weise eingeführt.

Die Einrichtung und/oder die Verbindungsöffnung sind jeweils vollständig oder in wenigstens einem Abschnitt hiervon als eine Hülse ausgestaltet oder weisen eine solche auf.

Eine Hülse ist in bestimmten erfindungsgemäßen Ausführungsformen ein Rohr oder ein Rohrabschnitt.

Hülsen können beispielsweise aus Kunststoff oder Metall hergestellt sein, mit den für diese Materialien bekannten Vorteilen.

In bestimmten Ausführungsformen der Erfindung ist die als Hülse oder Rohr ausgestaltete Verbindungsöffnung in der Dosiervorrichtung derart angeordnet, oder zu einer solchen Anordnung vorgesehen, dass sie sich vom Gehäuseabschnitt, in welchem die Verbindungsöffnung liegt, aus wenigstens 20 mm, vorzugsweise wenigstens 25 mm, weiter bevorzugt 30 oder 35 mm in das Innere der Dosiervorrichtung hinein und/oder in das Äußere hinaus erstreckt.

In manchen Ausführungsformen weist die als Hülse oder Rohr ausgestaltete Verbindungsöffnung in der Dosiervorrichtung eine Länge von 20 mm oder 25 mm oder 30 mm oder 35 mm auf. Die Hülse kann auch eine Länge zwischen diesen Werten oder kürzer als 20 mm oder länger als 35 mm aufweisen.

Diese in den beiden vorangegangenen Absätzen genannten Längen tragen vorteilhaft dazu bei, dass z. B. Desinfektionsflüssigkeit durch die Verbindungsöffnung nicht in das Innere der Dosiervorrichtung eintritt. Größere Längen sind in manchen erfindungsgemäßen Ausführungsformen hierzu nicht erforderlich.

In bestimmten Ausführungsformen der Erfindung ist die erfindungsgemäße Einrichtung angeordnet oder vorgesehen zu ihrer Anordnung, in welcher die erfindungsgemäße Einrichtung mit wenigstens einem Abschnitt hiervon innerhalb wenigstens eines Abschnitts der - beispielsweise als Hülse, oder nicht als Hülse ausgestalteten - Verbindungsöffnung relativ zu dieser verschiebbar ist.

In manchen erfindungsgemäßen Ausführungsformen werden die Materialien der erfindungsgemäßen Einrichtung und der die Verbindungsöffnung umgebenden Struktur derart ausgewählt, dass beide Elemente möglichst reibungsarm oder reibungsoptimiert gegeneinander verschiebbar sind. Entsprechende Werkstoffpaarungen sind dem Fachmann bekannt.

In bestimmten Ausführungsformen der Erfindung ist die erfindungsgemäße Einrichtung angeordnet oder vorgesehen zu ihrer Anordnung, um insgesamt oder mit wenigstens einem Abschnitt der Einrichtung koaxial - oder im Wesentlichen koaxial - innerhalb der Verbindungsöffnung verschiebbar zu sein.

Die erfindungsgemäße Behandlungsvorrichtung ist in manchen Ausführungsformen eine Blutbehandlungsvorrichtung, insbesondere eine Hämodialysevorrichtung, eine Hämofiltrationsvorrichtung oder eine Hämodiafiltrationsvorrichtung.

Das Verfahren zum Ermöglichen oder Durchführen einer Erkennung der Lage eines bewegbaren Abschnitts umfasst in bestimmten erfindungsgemäßen Ausführungsformen das Anordnen der erfindungsgemäßen Einrichtung oder eines Abschnitts hiervon innerhalb einer Verbindungsöffnung oder innerhalb eines Abschnitts der Verbindungsöffnung.

Bestimmte erfindungsgemäße Ausführungsformen weisen einen oder mehrere der im Folgenden genannten Vorteile auf.

Die vorliegende Erfindung stellt eine Einrichtung zum Führen eines Kabels in einer medizintechnischen Dosiervorrichtung bereit. Das Kabel kann bei der Erfüllung der Funktionsweise der Dosiervorrichtung vorteilhaft relativ zu dieser oder zu Abschnitten hiervon bewegt werden, ohne hierbei mechanisch belastet zu sein, beispielsweise im Bereich seiner Verbindung mit einem Sensor oder dem bewegbaren Abschnitt der Dosiervorrichtung. Das Kabel wird bei seiner Bewegung nicht beschädigt. Eine solche Beschädigung könnte ein Knicken, Beschädigen in der Nähe von heißen Maschinenteilen, Beschädigen an scharfen Kanten, Beschädigen durch Berühren von bewegten Teilen, Kabelbruch oder dergleichen sein. Derartige Beschädigungen werden durch die gemeinsame Führung des Kabels und der erfindungsgemäßen Einrichtung vorteilhaft verhindert.

Ferner kann beispielsweise ein Kabel oder Leiter, mittels welchem ein bewegtes Bauteil innerhalb der Dosiervorrichtung oder einer einem anderen Zweck dienenden Vorrichtung mit Spannung versorgt wird oder Signale abgibt, auch im Gebrauch unbeschadet nach außen geführt werden, um außerhalb der Vorrichtung beispielsweise mit einer Spannungsquelle oder einem Signalempfänger verbunden zu werden.

In bestimmten Ausführungsformen der Erfindung wird dabei ein Eindringen von Tropfwasser (z. B. durch einen Tropfwasserschutz nach IPX1), Desinfektionsmittel oder anderen Flüssigkeiten in das Innere der Dosiervorrichtung vorteilhaft verhindert. Diese vorteilhafte Wirkung ergibt sich in bestimmten erfindungsgemäßen Ausführungsformen aufgrund der Länge der Verbindungsöffnung, in manchen Ausführungsformen aufgrund eines schmal gehaltenen Spalts zwischen erfindungsgemäßer Einrichtung und Verbindungsöffnung (dies gilt vor allem dann, wenn die erfindungsgemäße Einrichtung in der Verbindungsöffnung koaxial zur dieser angeordnet ist). Damit können innenliegende elektrische Bauteile wie beispielsweise Sensoren vor Feuchtigkeit geschützt werden.

Die erfindungsgemäße Einrichtung kann ein kommerziell günstig erhältliches Rohr sein, oder, ebenfalls kostengünstig, als Spritzgussteil hergestellt werden.

Das Kabel kann in der erfindungsgemäßen Einrichtung einfach montiert werden, indem es durch diese hindurchgezogen oder eingesteckt wird. Im Inneren der erfindungsgemäßen Einrichtung ist es vor Beschädigung durch Einwirkung von außen auf das Kabel oder durch Hängenbleiben des Kabels an Komponenten der Dosiervorrichtung sowie durch mechanische Beanspruchung anderer Ursache vorteilhaft geschützt.

Aufwendige Kabelverlegungen können dabei vorteilhaft entfallen.

Das Kabel, das in der erfindungsgemäßen Einrichtung geführt wird, braucht sich bei Bewegen des bewegbaren Abschnitts der Dosiervorrichtung nicht - oder zumindest wenig - relativ zur erfindungsgemäßen Einrichtung zu bewegen, da das Kabel gemeinsam mit der Einrichtung geführt bzw. bewegt wird. Auch dies stellt einen Schutz vor mechanischer Beanspruchung des Kabels dar.

Die vorliegende Erfindung wird im Folgenden anhand der beigefügten Zeichnung, in welcher identische Bezugszeichen gleiche oder ähnliche Bauteile bezeichnen, exemplarisch erläutert. In den zum Teil vereinfachten Figuren gilt:
- **Fig. 1**: zeigt eine Schnittdarstellung einer erfindungsgemäßen Dosiervorrichtung mit einer erfindungsgemäßen Einrichtung zum Führen eines Kabels;
- **Fig. 2**: zeigt eine weitere Schnittdarstellung der Dosiervorrichtung der Fig. 1;
- **Fig. 3**: zeigt eine andere Ansicht des in Fig. 2 gezeigten Schnitts durch die erfindungsgemäße Dosiervorrichtung der Fig. 1; und
- **Fig. 4**: zeigt eine Seitenansicht der Dosiervorrichtung der Fig. 1.

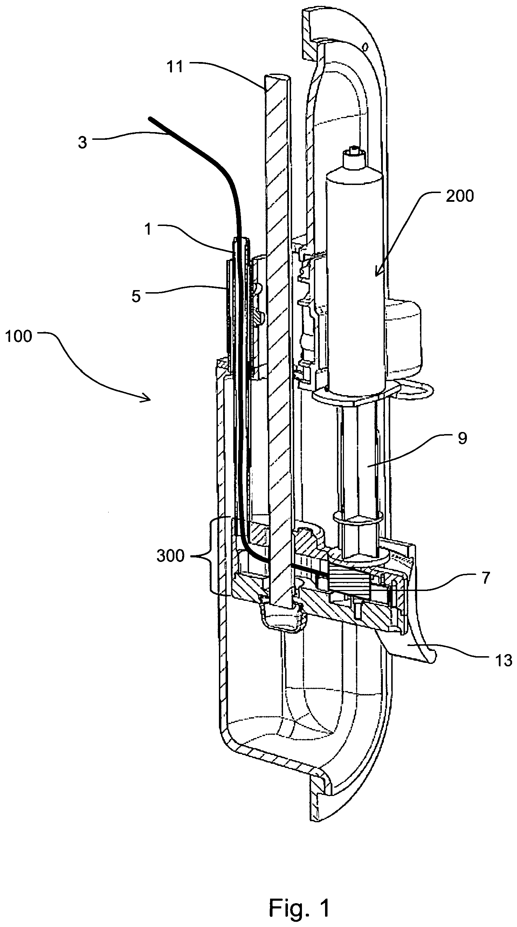

**Fig. 1** zeigt im Teil-Längsschnitt eine medizintechnische Dosiervorrichtung 100 mit einer erfindungsgemäßen Einrichtung 1 zum Führen eines Kabels 3.

Die Einrichtung 1 der Fig. 1 ist exemplarisch als eine erste Hülse oder Rohr dargestellt. Diese wird im oberen Bereich hiervon in einer als eine zweite Hülse oder Rohr ausgeführten Verbindungsöffnung 5 geführt. Die Verbindungsöffnung 5 verbindet den inneren Bereich der Dosiervorrichtung 100 mit dem Äußeren der Dosiervorrichtung 100.

Aufgrund der Länge der Verbindungsöffnung 5 einerseits und des schmalen Spalts zwischen Verbindungsöffnung 5 und Einrichtung 1 andererseits wird im Beispiel der Fig. 1 ein Eindringen von Tropfwasser, Desinfektionsmittel oder anderen Flüssigkeiten in das Innere der Dosiervorrichtung 100 vorteilhaft verhindert.

Das untere Ende (bezogen auf die Darstellung der Fig. 1) der erfindungsgemäßen Einrichtung 1 ist mit einem als Griffstück 300 ausgestalteten bewegbaren Abschnitt der Dosiervorrichtung 100 verbunden. Das untere Ende der Einrichtung 1 ist zum Inneren des Griffstücks 300 hin offen. Daher kann das Kabel 3, das mit einem Sensor 7 verbunden ist, welcher im Inneren des Griffstücks 300 angeordnet ist, von unten in die erfindungsgemäße Einrichtung 1 eingeführt werden. Das Kabel 3 kann gemeinsam mit der erfindungsgemäßen Einrichtung 1 durch die als zweite Hülse oder Rohr ausgeführte Verbindungsöffnung 5 nach außen geführt werden.

Der Sensor 7 ist in diesem Ausführungsbeispiel vorgesehen, um einen Spritzenstempel 9 einer Spritze 200 am Griffstück 300 zu detektieren. Damit können das Vorhandensein, die vorgesehene Lage der Spritze 200 in der Dosiervorrichtung 100 und/oder die Stellung des Spritzenstempels 9 relativ zur Spritze 200 oder zur Dosiervorrichtung 100 erfasst werden.

Eine Klemmvorrichtung 13 (von welcher in Fig. 1 nur ein Teil der an sich zweiteiligen Klemmvorrichtung dargestellt ist), fixiert den Spritzenstempel 9 im Griffstück 300.

Ein Stellelement 11 ist mit dem Griffstück 300 verbunden. Das Stellelement 11 ist vorgesehen, um das Griffstück 300 und damit den Spritzenstempel 9 mit dem Ziel der Abgabe von in der Spritze 200 enthaltenen Substanzen oder Lösungen zu bewegen. Das Stellelement 11 kann mit einem Motor, beispielsweise einem Schrittmotor, z. B. am oberen Ende des Stellelements 11, verbunden sein. Mittels geeigneter Steuerung des Motors kann eine vorgegebene Menge oder Rate an Medikamenten aus der Spritze abgegeben werden.

Bei einem Vorschub des Stellelements 11 (in Fig. 1 in eine Richtung nach oben) werden sowohl das Griffstück 300, der Spritzenstempel 9 als auch die Einrichtung 1 zum Führen des Kabels 3 gemeinsam mit dem Stellelement 11 mitbewegt.

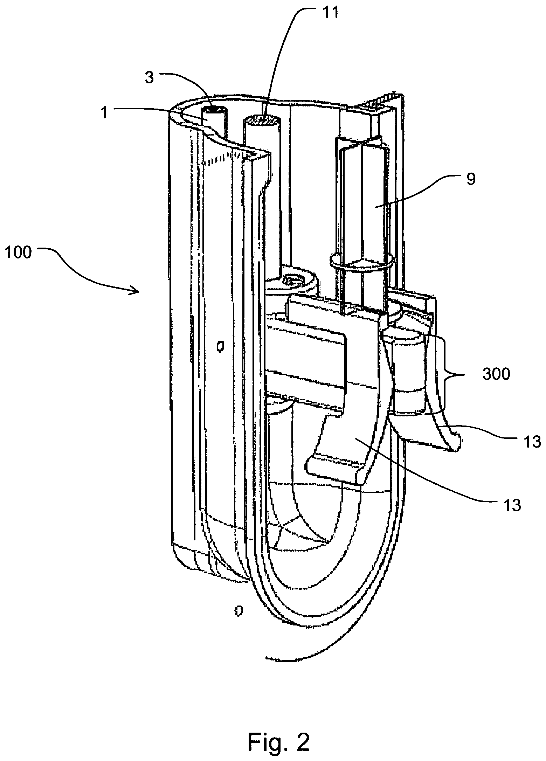

**Fig. 2** zeigt eine Querschnittsdarstellung der Dosiervorrichtung 100 der Fig. 1. Die Querschnittsebene liegt im Bereich des Spritzenstempels 9.

Der Querschnitt des Kabels 3 ist innerhalb der Einrichtung 1 dargestellt.

Oberhalb der Querschnittsebene schließt sich die Verbindungsöffnung 5 an, die in Fig. 2 jedoch nicht dargestellt ist.

Die Klemmvorrichtung 13 fixiert den Spritzenstempel 9 im Griffstück 300 von zwei Seiten.

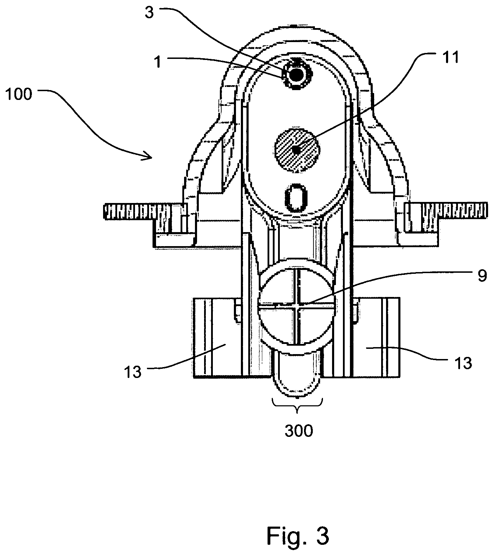

**Fig. 3** zeigt den Schnitt der erfindungsgemäßen Dosiervorrichtung 100 der Fig. 2 in einer zweidimensionalen Ansicht von oben (bezogen auf die Darstellung der Fig. 2). Die Querschnittsebene entspricht dem in Fig. 2 Dargestellten, was hier jedoch in einer Draufsicht gezeigt ist.

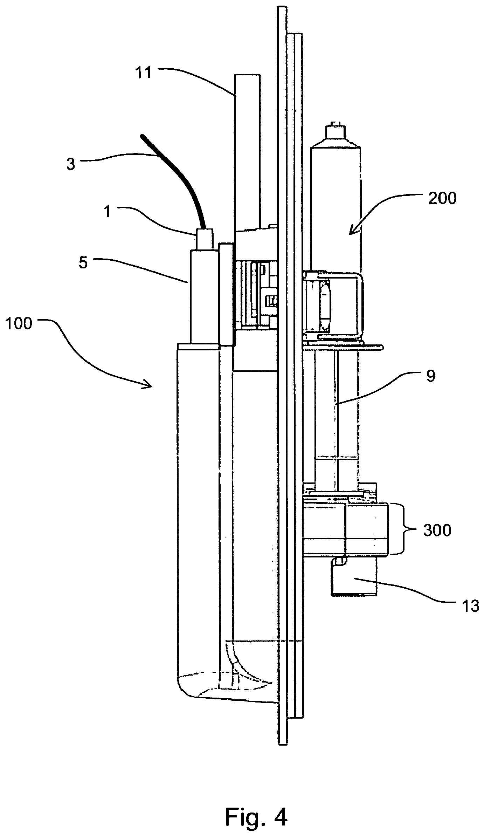

**Fig. 4** zeigt eine Seitenansicht der ungeschnittenen Dosiervorrichtung 100 der Fig. 1.

Aus der erfindungsgemäßen Einrichtung 1 heraus führt das Kabel 3 aus der Dosiervorrichtung 100 nach außen. Die erfindungsgemäße Einrichtung 1 ist wiederum durch die rohrförmige Verbindungsöffnung 5 eingesteckt und ragt aus dem Inneren der Dosiervorrichtung 100 heraus. Die erfindungsgemäße Einrichtung 1 ist in der Verbindungsöffnung 5 verschiebbar angeordnet.

Beim Gebrauch der Dosiervorrichtung 100 werden die folgenden Teile bzw. Abschnitte nach oben (bezogen auf die Darstellung der Fig. 4) bewegt: der Spritzenstempel 9, das Griffstück 300, die Klemmvorrichtung 13, die erfindungsgemäße Einrichtung 1 (geführt in der Verbindungsöffnung 5, die am Gehäuse der Dosiervorrichtung 100 fixiert ist) und das in der Einrichtung 1 geführte Kabel 3.

**Bezugszeichenliste**

| **Bezugszeichen** | **Beschreibung** |
|---|---|
| 100 | Medizintechnische Dosiervorrichtung |
| 200 | Spritze mit Stempel, als Aufnahmekammer |
| 300 | Griffstück, als beweglicher Abschnitt |
| 1 | Einrichtung zum Führen eines Kabels |
| 3 | Kabel |
| 5 | Verbindungsöffnung |
| 7 | Sensor |
| 9 | Spritzenstempel |
| 11 | Stellelement |
| 13 | Klemmvorrichtung |

## Patentansprüche

1. Dosiervorrichtung (100) für eine medizinische Verwendung, mit wenigstens einer Einrichtung (1) zum Führen wenigstens eines Kabels (3) oder Leiters,
wobei die Dosiervorrichtung (100) wenigstens einen bewegbaren Abschnitt aufweist, wobei die Einrichtung (1) mit dem wenigstens einen bewegbaren Abschnitt der Dosiervorrichtung (100) mitführbar verbunden ist,
wobei die Einrichtung (1) in eine Verbindungsöffnung (5) der Dosiervorrichtung (100) eingeführt ist,
wobei die Verbindungsöffnung (5) in einem Gehäuseabschnitt der Dosiervorrichtung (100) angeordnet ist und ein Inneres der Dosiervorrichtung (100) mit einem Äußeren der Dosiervorrichtung (100) verbindet;
**dadurch gekennzeichnet, dass**
die Einrichtung (1) und/oder die Verbindungsöffnung (5) als Hülse ausgestaltet ist bzw. sind oder eine Hülse aufweist bzw. aufweisen.

2. Dosiervorrichtung (100) nach Anspruch 1, wobei die Einrichtung (1) mit dem bewegbaren Abschnitt der Dosiervorrichtung (100) dauerhaft verbunden ist.

3. Dosiervorrichtung (100) nach Anspruch 1 oder 2, wobei das Kabel (3) mit einem in der Dosiervorrichtung (100) vorliegenden Sensor (7) verbunden ist, welcher vorgesehen ist, die Lage des wenigstens einen bewegbaren Abschnitts der Dosiervorrichtung (100) oder einer Aufnahmekammer für zu dosierende Substanzen oder Lösungen innerhalb der Dosiervorrichtung (100) zu erfassen, und/oder um das Vorhandensein der Aufnahmekammer innerhalb der Dosiervorrichtung (100) zu erfassen.

4. Dosiervorrichtung (100) nach einem der vorangegangenen Ansprüche, wobei die als Hülse ausgestaltete Verbindungsöffnung (5) in der Dosiervorrichtung (100) angeordnet ist, oder zu einer solchen Anordnung vorgesehen ist, um sich vom Gehäuseabschnitt aus, in welchem die Verbindungsöffnung (5) liegt, wenigstens 20 mm, vorzugsweise wenigstens 25 mm, weiter bevorzugt 30 oder 35 mm in das Innere der Dosiervorrichtung (100) hinein und/oder in das Äußere hinaus zu erstrecken.

5. Dosiervorrichtung (100) nach einem der vorangegangenen Ansprüche, wobei die Einrichtung (1) angeordnet ist, um in oder mit wenigstens einem Abschnitt hiervon innerhalb wenigstens eines Abschnitts der Verbindungsöffnung (5) und relativ zu dieser oder einem Abschnitt hiervon verschiebbar zu sein.

6. Dosiervorrichtung (100) nach Anspruch 5, wobei die Einrichtung (1) angeordnet ist, um mit ihrem wenigstens einen Abschnitt koaxial oder im Wesentlichen koaxial innerhalb der Verbindungsöffnung (5) verschiebbar zu sein.

7. Behandlungsvorrichtung, insbesondere Blutbehandlungsvorrichtung, insbesondere vorgesehen und ausgestaltet zum Durchführen einer Hämodialyse, Hämofiltration oder Hämodiafiltration an einem Patienten, aufweisend oder verbunden mit wenigstens einer Dosiervorrichtung (100) gemäß einem der Ansprüche 1 bis 6.

## Claims

1. A dosing apparatus (100) for medical use, comprising at least one device (1) for guiding at least one cable (3) or conductor,
wherein the dosing apparatus (100) comprises at least one movable section, wherein the device (1) is connected to the at least one movable section of the dosing apparatus (100) so as to move with it,
wherein the device (1) is introduced within a connection aperture (5) of the dosing apparatus (100),
wherein the connection aperture (5) is arranged in a housing section of the dosing apparatus (100) and connects an interior of the dosing apparatus (100) with an exterior of the dosing apparatus (100);
**characterized in that**
the device (1) and/or the connection aperture (5) is/are configured as sleeve or comprise(s) one sleeve.

2. The dosing apparatus (100) according to claim 1, wherein the device (1) is permanently connected to the movable section of the dosing apparatus (100).

3. The dosing apparatus (100) according to claim 1 or 2, wherein the cable (3) is connected to a sensor (7) positioned in the dosing apparatus (100), which is provided to detect the position of the at least one movable section of the dosing apparatus (100) or of a receiving chamber for substances or solutions to be dosed within the dosing apparatus (100), and/or to detect the presence of the receiving chamber within the dosing apparatus (100).

4. The dosing apparatus (100) according to anyone of the preceding claims, wherein the connection aperture (5) configured as a sleeve is arranged in the dosing apparatus (100), or is provided for such an arrangement, so as to extend at least 20 mm, preferably at least 25 mm, more preferably 30 or 35 mm, from the housing section in which the connection aperture (5) lies to the interior and/or to the exterior of the dosing apparatus (100).

5. The dosing apparatus (100) according to anyone of the preceding claims, wherein the device (1) is arranged to be movable in or with at least one section thereof, within at least one section of the connection aperture (5) and relative to this or to a section thereof.

6. The dosing apparatus (100) according to claim 5, wherein the device (1) is arranged to be coaxially or essentially coaxially movable with its at least one section within the connection aperture (5).

7. A treatment device, especially a blood treatment device provided and designed to execute a hemodialysis, a hemofiltration or a hemodiafiltration of a patient, comprising or connected with at least one dosing apparatus (100) according to anyone of claims 1 to 6.

## Revendications

1. Un appareil de dosage (100) pour une utilisation médicale, comprenant au moins un dispositif (1) destiné à guider au moins un câble (3) ou un conducteur,
où l'appareil de dosage (100) comprend au moins une partie mobile, où le dispositif (1) est relié à au moins une partie mobile de l'appareil de dosage (100) de façon à être conjointement entrainable,
où le dispositif (1) est introduit dans une ouverture de raccordement (5) de l'appareil de dosage (100),
où l'ouverture de raccordement (5) est agencée dans une partie du boîtier de l'appareil de dosage (100) et relie un intérieur de l'appareil de dosage (100) à un extérieur de l'appareil de dosage (100);
**caractérisé en ce que**
le dispositif (1) et/ou l'ouverture de raccordement (5) est/sont configuré(s) sous forme de gaine ou comprend/comprennent une gaine.

2. L'appareil de dosage (100) selon la première revendication, où le dispositif (1) est relié en permanence à la partie mobile de l'appareil de dosage (100).

3. L'appareil de dosage (100) selon la première ou la seconde revendication, où le câble (3) est relié à un capteur (7) présent dans l'appareil de dosage (100), lequel est prévu pour détecter la position d'au moins une partie mobile de l'appareil de dosage (100) ou d'une chambre de réception pour les substances ou les solutions à doser à l'intérieur de l'appareil de dosage (100), et/ou pour détecter la présence de la chambre de réception à l'intérieur de l'appareil de dosage (100).

4. L'appareil de dosage (100) selon l'une quelconque des revendications précédentes, où l'ouverture de raccordement (5), configurée sous forme de gaine, est agencée dans l'appareil de dosage (100), ou est prévue pour un tel agencement, de façon à s'étendre d'au moins 20 mm, de préférence d'au moins 25 mm, mieux encore de 30 ou 35 mm, vers l'intérieur et/ou vers l'extérieur de l'appareil de dosage (100) depuis la partie du boîtier dans laquelle se trouve l'ouverture de raccordement (5).

5. L'appareil de dosage (100) selon l'une des revendications précédentes, où le dispositif (1) est agencé de façon à pouvoir coulisser, dans ou avec au moins une partie de ce dernier, à l'intérieur d'au moins une partie de l'ouverture de raccordement (5) et relativement à celle-ci ou à une partie de celle-ci.

6. L'appareil de dosage (100) selon la revendication 5, où le dispositif (1) est agencé de façon à pouvoir coulisser coaxialement ou essentiellement coaxialement, avec au moins sa partie, à l'intérieur de l'ouverture de raccordement (5).

7. L'appareil de traitement, notamment un appareil de traitement du sang, prévu et conçu notamment pour effectuer une hémodialyse, une hémofiltration ou une hémodiafiltration à un patient, comprenant ou étant relié à au moins un appareil de dosage (100) selon une des revendications 1 à 6.
